(19) Europäisches Patentamt
European Patent Office
Office européen des brevets

(11) Veröffentlichungsnummer: **0 014 252**
**B1**

(12) # EUROPÄISCHE PATENTSCHRIFT

(45) Veröffentlichungstag der Patentschrift: **12.01.83**

(51) Int. Cl.³: **C 12 Q 1/44**

(21) Anmeldenummer: **79105154.3**

(22) Anmeldetag: **13.12.79**

(54) **Reagens zur Lipasebestimmung und Verfahren zu seiner Herstellung.**

(30) Priorität: **05.02.79 DE 2904305**

(43) Veröffentlichungstag der Anmeldung: **20.08.80 Patentblatt 80/17**

(45) Bekanntmachung des Hinweises auf die Patenterteilung: **12.01.83 Patentblatt 83/2**

(84) Benannte Vertragsstaaten: **AT BE CH DE FR GB IT LU NL SE**

(56) Entgegenhaltungen:
**DE-A-1 961 983**
**DE-A-2 719 704**
**US-A-3 917 515**
**CHEMICAL ABSTRACTS, Band 84, Nr. 11, 15. März 1976, Zusammenfassung nr. 70752e, Seite 153, Columbus, Ohio, US, J. RATHELOT et al.: a"Studies on the effect of bile salt an colipase on enzymatic lipolysis. Improved method for the determination of pancreatic lipase and colipase" & Biochemie, 1975, 57(10), 1117–22**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Neumann, Ulrich Dr., Glückauf Strasse 3A, D-8123 Peissenberg (DE)**
Erfinder: **Knitsch, Karl-Wolfgang Dr., Machtlfing Nr. 77, D-8131 Andechs (DE)**
Erfinder: **Ziegenhorn, Joachim Dr., Ina-Seidel-weg 1, D-8130 Starnberg (DE)**
Erfinder: **Röder, Albert Dr., Bahnhofstrasse 41, D-8124 Seeshaupt (DE)**
Erfinder: **Zwez, Werner Dr., Tiefentalweg 8, D-8124 Seeshaupt (DE)**
Erfinder: **Krämer, Werner, Zugspitzstrasse 16, D-8131 Bernried (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

**EP 0 014 252 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Reagens zur Lipasebestimmung und Verfahren zu seiner Herstellung

Die Erfindung betrifft ein Reagens für die Bestimmung der Lipase, insbesondere im turbidimetrischen Test, und dessen Herstellung.

Für die Diagnostik der Pankreaserkrankung besitzt die Bestimmung der Enzyme Amylase (E.C. 3.2.1.1) und Lipase (E.C. 3.1.1.3) eine hohe Aussagekraft. Bei akuter Pankreatitis kommt es innerhalb von wenigen Stunden zu einem mitunter sehr massiven Anstieg beider Enzyme im Serum. Bei intakter Nierenfunktion wird jedoch die Amylase wegen ihres geringen Molekulargewichts schneller wieder ausgeschieden, während die Lipaseaktivität im Serum längere Zeit erhöht bleibt. Eine sichere Diagnose der Pankreatitis ist durch Bestimmung beider Enzyme zu erhalten.

Die Lipase spaltet primär die α-Esterbindung eines Triglycerids, mit bevorzugt langkettigen Fettsäureresten zum Diglycerid und der freien Fettsäure. Die weitere Umsetzung zum Monoglycerid verläuft erheblich langsamer.

Während die üblichen durch Enzyme katalysierten Reaktionen in der wässrigen Phase verlaufen, wirkt die Lipase nur an der Grenzfläche Öltröpfchen/Wasser. Hierdurch unterscheidet sie sich von den Esterasen, die in der wässrigen Phase reagieren. Neben den chemischen Parametern wird daher die Kinetik durch die Beschaffenheit der Oberfläche des Substrats in hohem Masse beeinflusst.

Titrimetrische Lipase-Bestimmungsmethoden sind bekannt. Sie konnten jedoch wegen der zum Teil schwierigen Handhabbarkeit, langen Reaktionsdauer und des hohen Probenbedarfs nur in begrenztem Masse Eingang in das klinisch-chemische Routinelabor finden.

Die wesentlichen Nachteile der titrimetrischen Bestimmungsmethode, nämlich der hohe Probenbedarf und die langen Inkubationszeiten, werden bei den turbidimetrischen Methoden vermieden, bei denen die Trübungsaufhellung einer Triglycerid/Wasser-Emulsion photometrisch verfolgt wird. Ein erhebliches Problem bei der turbidimetrischen Bestimmung ist jedoch die Herstellung einer Emulsion mit stets zuverlässig gleicher Tröpfchengrösse. Dies ist von besonderer Bedeutung, da die Kinetik der Reaktion von der Grösse der Triglyceridtröpfchen stark beeinflusst wird. Aus der DE-B 1 961 983 ist zwar bereits eine Olivenöltrockenemulsion bekannt, welche für die titrimetrische Bestimmung der Lipaseaktivität geeignet ist. Bei dieser Trockenemulsion ist jedoch der Triglyceridgehalt für eine photometrische Bestimmung der Trübungsaufhellung wegen der hohen Lichtabsorption nicht geeignet. Bei Verdünnung dieses Reagens mit dem Ziel, es auch für die turbidimetrische kinetische Lipasebestimmung geeignet zu machen, verschieben sich jedoch die Parameter derart, dass man keine Reaktion nullter Ordnung mehr erhält.

Eine Aufgabe der Erfindung ist daher die Schaffung eines insbesondere für die turbidimetrische bzw. nephelometrische Aktivitätsbestimmung der Lipase geeignetes Reagens zu schaffen, welches in Trockenform vorliegt und durch einfaches Versetzen mit Wasser in eine Emulsion mit reproduzierbar gleicher Tröpfchengrösse überführt werden kann. Ein weiteres Ziel der Erfindung ist die Schaffung eines derartigen Reagens, bei welchem sich die Tröpfchengrösse der rekonstituierten Emulsion durch die quantitative Zusammensetzung des Trockenreagens regeln lässt und weitgehend unabhängig ist von der für die Herstellung des Trockenreagens eingesetzten Triglyceridemulsion. Hierdurch soll vermieden werden, dass der Anwender sich die Triglyceridemulsion unmittelbar vor der Durchführung des Tests selbst herstellen muss. Da nämlich die Beschaffenheit der Substratoberfläche in starkem Masse die Aktivität des Enzyms bestimmt (Clin. Chem. 23, 522–53, 1977), ist für zuverlässige Resultate eine stets gleichbleibende Substratoberfläche, auch hinsichtlich der Tröpfchengrösse, erforderlich. Die Emulsionsqualität ist jedoch sehr stark von der Technik der Herstellung abhängig und erfahrungsgemäss selbst bei Einhaltung bestimmter Vorschriften bei Durchführung durch unterschiedliche Personen unterschiedlich, so dass erhebliche Abweichungen in den gemessenen Aktivitätswerten auftreten. Auch diese Schwierigkeit soll durch die Erfindung beseitigt werden.

Gelöst wird diese Aufgabe durch ein rekonstituierbares Trockenreagens zur insbesondere turbidimetrischen Bestimmung der Lipase, welches bei Wasserzugabe eine Emulsion bildet, mit einem Gehalt an Substratöl, Schutzkolloid, Emulgator und Aktivator, welches dadurch gekennzeichnet ist, dass es

0,2 bis 10 Gew.-% flüssiges Triglycerid,
20 bis 90 Gew.-% Schutzkolloid,
5 bis 60 Gew.-% Gallensäurealkalisalz,
0,001 bis 0,1 Gew.-% Colipase,
0,1 bis 2,0 Gew.-% Konservierungsmittel,
gegebenenfalls 5 bis 20 Gew.-% Harnstoff,
3 bis 50 Gew.-% Puffersubstanz für pH 6,0 bis 10,5 und
0,5 bis 5 Gew.-% Aktivator

enthält.

Überraschenderweise hat sich gezeigt, dass bei der erfindungsgemässen Zusammensetzung eines Trockenreagens bei gleichbleibender quantitativer Zusammensetzung zuverlässig stets gleiche Tröpfchengrösse der rekonstituierten Emulsion erhalten wird und ausserdem auch die Beschaffenheit der Substratoberfläche reproduzierbar so beschaffen ist, dass stets eine hohe Reaktionsgeschwindigkeit und ein Reaktionsablauf nullter Ordnung erzielt wird, was Voraussetzung ist für eine praxisgerechte turbidimetrische, kinetische Aktivitätsbestimmung.

Durch Änderung der quantitativen Zusammensetzung im Rahmen der oben für die einzelnen Bestandteile des Reagens angegebenen Variationsbreite lässt sich unabhängig von der Herstellung eine bestimmte Tröpfchengrösse der rekonstituierten Emulsion festlegen. Da durch den Gehalt an Triglycerid die Anfangsextinktion des Reagensansatzes bedingt wird, ist in der Regel von einer bestimmten gewünschten Triglyceridkonzentration auszugehen, und die Mengen der übrigen Bestandteile können dann den jeweiligen Gegebenheiten hinsichtlich der angestrebten Tröpfchengrösse angepasst werden.

Als Triglycerid eignen sich an sich sowohl natürliche als auch synthetische Triglyceride mit Fettsäureresten zwischen etwa 4 und etwa 22 C-Atomen. Beispielsweise erwies sich auch das Tributyrin als geeignet. Bevorzugt werden jedoch Triglyceride mit längerkettigen ungesättigten Fettsäureresten, insbesondere Triglyceride, deren Fettsäurereste 8 bis 20 C-Atome und 1 bis 8, vorzugsweise 1 bis 3 Kohlenstoff-Kohlenstoff-Doppelbindungen aufweisen. Aufgrund seiner leichten Zugänglichkeit wird Triolein besonders bevorzugt, auch Olivenöl ist sehr gut geeignet. Die bevorzugte Triglyceridmenge beträgt 0,2 bis 2,0 Gew.-%.

Als Schutzkolloid kommen die dem Chemiker hierfür bekannten Substanzen in Betracht, wie Polyhydroxyverbindungen, Serumalbumin, Polyvinylpyrrolidon, feste Polyäthylenoxide und dergleichen. Bevorzugt werden Polyhydroxyverbindungen, insbesondere monomere oder polymere Pentose oder Hexose mit 1 bis 10 Pentosebzw. Hexose-Einheiten im Molekül oder/und bei Zimmertemperatur fester Polyäthylenglykol. Bevorzugte Beispiele von geeigneten Polyhydroxyverbindungen sind Mannit und ähnliche Zuckeralkohole, Oligosaccharid der Glucose, Mannose, Maltoheptaose, Polyäthylenglykol mit einem mittleren Molekulargewicht zwischen 3500 und 7000 u.ä. Andere verwendbare Schutzkolloide sind z.B. Aminosäuren, wie Alanin, Pflanzengummis, wie Gummi arabicum usw. Die bevorzugte Menge an Schutzkolloid bzw. einer Mischung von Schutzkolloiden beträgt 50 bis 70 Gew.-%. Als besonders geeignet erwies sich eine Mischung von Zuckeralkohol und Polyalkylenglykol.

Als Gallensäure kommen die bekannten oberflächenaktiven Gallensäuren, wie Cholsäure, Taurocholsäure, Desoxycholsäure, Taurodesoxycholsäure, Glycodesoxycholsäure bzw. deren Alkalisalze, insbesondere das Natriumsalz, in Betracht. Die bevorzugte Menge beträgt 10 bis 15 Gew.-%.

Ein weiterer wesentlicher Bestandteil des erfindungsgemässen Reagens ist Colipase. Besonders geeignet ist eine von Verunreinigungen freie Colipase. Die bevorzugte Menge beträgt 0,003 bis 0,01 Gew.-%.

Als Konservierungsmittel werden im Rahmen der Erfindung solche eingesetzt, die die enzymatische Aktivität der zu bestimmenden Lipase nicht beeinträchtigen. Besonders geeignet sind Alkaliazide, insbesondere Natriumazid. Auch andere Konservierungsmittel, wie z.B. Thiozid und andere schwefelhaltige Konservierungsmittel sind jedoch ebenfalls geeignet. Die bevorzugte Menge an Konservierungsmittel beträgt 0,2 bis 0,7 Gew.%.

Das erfindungsgemässe Reagens enthält ferner Harnstoff, bevorzugt 10 bis 15 Gew.-%.

Als Puffersubstanz eignen sich alle bekannten Puffer, welche in der Lage sind, im Rahmen des erfindungsgemässen Reagens einen pH-Wert zwischen 6,0 und 10,5 einzustellen. Der bevorzugte pH-Wertbereich liegt zwischen 8,3 und 9,5. Beispiele für geeignete Puffer sind Diäthanolaminpuffer, Triäthanolaminpuffer, Tris-Puffer und Goodpuffer, wie Hepes-Puffer (gut geeignet zum Zusatz vor der Lypophilisation), Taps-Puffer und Bicine. Besonders bevorzugt wird Tris-Puffer. Die bevorzugte Puffersubstanzmenge liegt zwischen 3 und 10 Gew.-%. Ein weiterer Aktivator sind Calciumionen. Da diese mit Desoxycholsäure unlösliche Verbindungen eingehen, wird bei Gegenwart von Calcium als Gallensäure Taurodesoxycholsäure bevorzugt, da diese höhere Calciumkonzentrationen im Bereich von 1 bis 5 mMol zulässt.

Ausser den genannten wesentlichen Bestandteilen kann das erfindungsgemässe Reagens auch für bestimmte Zwecke noch inerte Zusatzstoffe (Füllstoffe) enthalten, welche die Handhabung erleichtern.

Schliesslich enthält das erfindungsgemässe Reagens auch Aktivator für die Lipase. Lipaseaktivatoren sind bekannt. Bevorzugt werden Chloride, insbesondere Natriumchlorid, aber auch andere Alkali- oder Erdalkalichloride, soweit sie nicht zur Bildung unlöslicher Verbindungen mit anderen Bestandteilen des erfindungsgemässen Reagens oder der Probe führen. Auch Magnesiumionen wirken aktivierend.

Über die Wirkungsweise der einzelnen Bestandteile des erfindungsgemässen Reagens im Rahmen der Erfindung besteht nicht in jeder Hinsicht Klarheit. Es wird jedoch angenommen, dass die Colipase eine durch den hohen Gehalt an Gallensäuresalz, insbesondere an Desoxycholat, Taurodesoxycholat oder Glykodesoxycholat bewirkte Hemmung des Enzyms aufhebt und die Linearität des Reaktionsablaufs verbessert. Harnstoff scheint einen Einfluss auf die Wasser/Lipid-Oberfläche der Emulsion auszuüben und diese zu stabilisieren.

Das erfindungsgemässe Trockenreagens wird durch Lyophilisierung einer nach üblichen Methoden hergestellten Emulsion der Bestandteile hergestellt. Dabei werden vorzugsweise Puffersubstanz, Harnstoff und gegebenenfalls auch ein Teil des Schutzkolloids und des Konservierungsmittels sowie gegebenenfalls des Aktivators erst nach der Lyophilisierung der «Trockenemulsion» zugesetzt. Die der Lyophilisierung unterworfene Emulsion lässt sich nach üblichen Methoden herstellen, beispielsweise durch Einbringen aller Bestandteile in das wässrige Lösungsmittel, Emulgieren durch übliche Methoden, wie Ultraschall, Kolloidmühle und dergleichen und anschliessend Einfrieren bei etwa – 40 °C und Trocknen im Vakuum unter den üblichen Bedingungen, also etwa $10^{-1}$ bis $10^{-4}$ torr.

Bei der Lyophilisierung müssen Gallensäuresalz, Colipase, mindestens 20 Gew.-% Schutzkolloid, mindestens ein Teil des Konservierungsmit-

tels und gegebenenfalls Aktivator zugegeben sein. Ein bevorzugtes Verfahren zur Herstellung der für die Lyophilisierung bestimmten wässrigen Emulsion besteht darin, dass man die erwähnten Bestandteile, ausgenommen das Triglycerid, in Wasser auflöst und in die Lösung dann eine Lösung des Triglycerids in einem flüchtigen organischen Lösungsmittel in feinem Strahl unter Rühren einspritzt. Als flüchtige organische Lösungsmittel eignen sich dabei insbesondere aliphatische Alkohole und Ketone mit 1 bis 4 Kohlenstoffatomen.

Die restlichen Bestandteile des erfindungsgemässen Reagens, nämlich Puffersubstanz, Harnstoff und gegebenenfalls weiteres Schutzkolloid, Konservierungsmittel und Aktivator, können dem Lyophilisat unmittelbar nach seiner Herstellung oder erst später zugegeben werden.

Wie bereits erwähnt, wurde überraschenderweise gefunden, dass das erfindungsgemässe Reagens nach der Rekonstitution eine Extinktion oder eine Extinktionsänderung pro Zeiteinheit ergibt, die weitgehend von der Extinktion der Ausgangsemulsion und der Herstellung des Reagens unabhängig ist. Ausserdem ist die Extinktionskonstanz der rekonstituierten Emulsion überraschend gut.

Die Gallensäuresalze werden vorzugsweise von Verunreinigungen, wie Abbauprodukten und dergleichen, gereinigt, beispielsweise durch Extraktion mit n-Butanol unter alkalischen Bedingungen, Umkristallisation aus Alkohol/Aceton und ähnlichen Reinigungsmethoden.

Diese überraschende Eigenschaft des erfindungsgemässen Reagens wurde durch Bestimmung der Tröpfchengrössenverteilung vor und nach der Lyophilisation mit Hilfe eines Coulter-Counter untersucht. Mit diesem Gerät ist es möglich, die prozentuale Verteilung von Teilchen des Durchmessers von 480 bis 16 000 nm in 100- bis 1000 nm-Abständen zu bestimmen. Dabei zeigte es sich, dass die Tröpfchenverteilung vor der Lyophilisation von Ansatz zu Ansatz sehr stark schwankte, nach der Lyophilisation und gegebenenfalls weiteren Verfahrensschritten, wie Zumischung der anderen Bestandteile des Reagens und dergleichen, stets ein gut übereinstimmendes Verteilungsmuster aufwies (Einstellung eines thermodynamischen Gleichgewichts). Die so hergestellte Trockenemulsion zeichnet sich weiter dadurch aus, dass auch bei thermischer Belastung (3 Wochen/35 °C) eine sehr gute Konstanz der Tröpfchenverteilung gewährleistet ist. Das Maximum der Tröpfchenverteilung wurde bei 500 bis 1000 nm gefunden. Sie eignen sich daher besonders für die photometrische Verfolgung der Trübungsaufhellung bei 340 oder 365 nm. Bei einer Teilchengrösse wesentlich unter 340 nm ist ein Abbau der Triglyceridtröpfchen nicht mehr feststellbar, grosse Tröpfchen hingegen sind kein ideales Substrat der Lipase.

Ein wesentlicher Vorteil des erfindungsgemässen Reagens liegt darin, dass es sowohl bei humaner Pankreaslipase als auch bei Schweinepankreaslipase gleiche Aktivitätsänderung pro Zeiteinheit aufweist. Da im titrimetrischen Test nach Rick diese beiden Lipasen ebenfalls gleiche Aktivitätskonzentrationen zeigen, ist es möglich, zur Kalibrierung einen schweinepankreashaltigen Standard einzusetzen.

Ein weiterer wesentlicher Vorteil des erfindungsgemässen Reagens ist das Fehlen einer Lag-Phase. Es ist bekannt, dass beim turbidimetrischen Lipasetest erst nach einiger Zeit ein Reaktionsablauf nullter Ordnung auftritt. Ein derartiger Reaktionsablauf ist aber für die exakte Bestimmung der Aktivitätskonzentration eines Enzyms unbedingt erforderlich (vgl. H.U. Bergmeyer, Grundlagen der enzymatischen Analyse, 1979, 58ff). Die Zeit bis zur Erreichung dieses Reaktionsablaufs wird als Lag-Phase bezeichnet.

Die folgenden Beispiele erläutern die Erfindung weiter in Zusammenhang mit der beigefügten Zeichnung. Die Figuren der Zeichnung zeigen in graphischer Darstellung die am Coulter-Counter bei verschiedenen Beispielen und beschriebenen Versuchen erhaltene Tröpfchengrössenverteilung der Emulsion.

Beispiel 1

0,06 g $CACl_2$, 22,72 g Natriumdesoxycholat, 25,35 g Polyäthylenglykol, MG 4000, 1,1 g Natriumazid, 0,02 g Colipase und 50,8 Teile Mannit werden in 1,0 l destilliertem Wasser gelöst. In die erhaltene Lösung werden unter Rühren 1,42 g Triolein, gelöst in 32 ml n-Propanol mit einem Druck von 2 bar durch eine Düse von 1,5 mm Durchmesser eingespritzt. Die so erhaltene Trioleinemulsion wird bei −40 °C eingefroren und durch Lyophilisieren getrocknet.

8,95 g Natriumdesoxycholat, 7,25 g Tris, 1,20 g Tris. HCl, 7,5 g Polyäthylenglykol, MG 4000, 1,97 g NaCl, 16,2 g Harnstoff und 56,9 g Mannit werden trocken miteinander vermischt. 2 Gew.-Teile des so erhaltenen Puffergemisches werden mit 1 Gew.-Teil des Lyophilisats mittels Spiralmischer zu einem homogenen Reagens vermischt.

Zur Durchführung der Lipasebestimmung werden 150 mg dieses Reagens mit 2,5 ml Wasser aufgenommen, mit 0,1 ml Probe vermischt und photometrisch bei 365 nm bei 25 °C gegen Wässer gemessen. Als Probe eignen sich beispielsweise Serum, Duodenalsaft, Urin und andere Körperflüssigkeiten.

Die Richtigkeit der Methode wurde durch Vergleich mit der titrimetrischen Methode nach Rick durchgeführt. Bei einer Untersuchung von 60 Humanseren unterschiedlicher Lipaseaktivität ergaben sich folgende Daten:

Korrelation: (x = Rick; y = erfindungsgemässer Test)

y = 0,93. x − 20; r =0,96.

Die Linearität des Tests blieb bis ca. 1300 Rick-Einheiten erhalten.

1 Rick-Einheit = 1μMol FFA/Minute = 1U FFA = freigesetzte Fettsäuren.

Beispiel 2

1,500 g Mannit, 0,900 g Polyäthylenglykol MG 4000, 0,524 g Natriumdesoxycholat, 1,5 mg Colipase und 8 mg Natriumazid werden in 50 ml destilliertem Wasser gelöst. Zu dieser Lösung werden durch eine feine Düse 150 mg Triolein, gelöst in 5,0 ml Propanol, unter Rühren eingespritzt. Die erhaltene Emulsion wird bei −40 °C eingefroren und lyophilisiert.

3,50 g Harnstoff, 3,73 g Natriumdesoxycholat, 0,4 g NaCl, 0,10 g Natriumazid, 2 mg Calciumcarbonat, 1,355 g Tris und 0,200 g Tris. HCl werden innig miteinander gemischt. Hierzu wird das zerkleinerte Trockenlyophilisat gegeben und homogen vermischt.

Die komplette Mischung = 8,219 g, enthält:

| | |
|---|---|
| Mannit | 1,500 g |
| Polyäthylenglykol | 0,900 g Summe = 2,4 g = 29,2 Gew.-% |
| Natriumdesoxycholat | 4,254 g = 51,7 Gew.-% |
| Colipase | 1,5 mg |
| Natriumazid | 9 mg |
| Tris | 1,355 g |
| Tris. HCl | 0,200 g Summe = 1,555 = 18,9 Gew.-% |
| Harnstoff | 3,5 g = 42,5 Gew.-% |

37 mg dieser Mischung werden in 2,5 ml destilliertem Wasser gelöst und mit 200 µl Probe (Serum) bei 340 nm und 25 oder 30 °C gegen Wasser oder Luft photometrisch gemessen. Aus der Extinktionsdifferenz/min erfolgt die Bestimmung der Lipaseaktivität.

Die Verteilung der Tröpfchengrösse nach Rekonstitution sowohl in frisch hergestelltem Zustand als auch nach drei Wochen Belastung bei 35 °C in trockenem Zustand, gemessen am Coulter-Counter, zeigt Fig. 1 der Zeichnung.

Beispiel 3

8,41 g Rinderserumalbumin, 12,2 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17,4 g Mannit, 4 g Polyäthylenglykol fest, 7 g Harnstoff, 7,47 g Natriumdesoxycholat, 0,82 g NaCl, 0,2 g Natriumazid, 2,71 g Tris und 0,4 g Tris. HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Beispiel 4

8,41 g Alanin, 1,22 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17.4 Mannit, 4 g Polyäthylenglykol fest, 7 g Harnstoff, 7,47 g Natriumdesoxycholat, 0,82 NaCl, 0,2 g Natriumazid, 2,71 g Tris und 0,4 g Tris. HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Beispiel 5

4,205 g Polyäthylenglykol MG 4000, 4,205 g Rinderserumalbumin, 1,22 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17,4 g Mannit, 4 g Polyäthylenglykol MG 4000, 7 g Harnstoff, 7,47 g Natriumdesoxycholat, 0,82 g NaCl, 0,2 g Natriumazid, 2,71 g Tris und 0,4 g Tris.HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Beispiel 6

6,73 g Polyäthylenglykol (Polywachs 4000), 1,68 g Polyvinylpyrrolidon, 1,22 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17,4 g Mannit, 4 g Polyäthylenglykol, 7 g Harnstoff, 7,47 g Natriumdesoxycholat, 0,82 g NaCl, 0,2 g Natriumazid, 2,71 g Tris und 0,4 g Tris.Hcl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Beispiel 7

(Vergleichsbeispiel; zu wenig Gallensäuresalz)

4,69 g Mannit, 4,69 g Polyäthylenglykol (Polywachs 4000), 0,38 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17,4 g Mannit, 4 g Polyäthylenglykol MG 4000, 7 g Harnstoff, 0,82 g NaCl, 0,2 g Natriumazid, 2,71 g Tris und 0,4 g Tris.HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Die Verteilung der Tröpfchengrösse mit und ohne Belastung (siehe Beispiel 2) zeigt Fig. 2 der Zeichnung.

Beispiel 8 (Vergleichsbeispiel)

3,76 g Mannit, 5,63 g Polyäthylenglykol MG 4000, 0,19 g Natriumdesoxycholat, 0,02 g Natriumazid und 0,034 g Colipase werden in 100 ml destilliertem Wasser gelöst. Unter Rühren wird eine Lösung von 3,5 g Triolein in 7 ml Propanol unter Druck in diese Lösung eingespritzt. Die so entstandene Emulsion wird bei −40 °C eingefroren und lyophilisiert.

17,4 g Mannit, 4 g Polyäthylenglykol MG 4000, 7 g Harnstoff, 0,82 g NaCl, 0,2 g Natriumazid, 2,71 g Tris und 0,4 g Tris.HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (365) nmHg photometrisch verfolgt.

Die Verteilung der Tröpfchengrösse mit und ohne Belastung (siehe Beispiel 2) zeigt Fig. 3 der Zeichnung.

Beispiel 9

8,88 g Mannit, 0,86 g Natriumdesoxycholat, 0,1 g Natriumazid und 0,034 g Colipase werden in 100 ml destilliertem Wasser gelöst. Unter Rühren wird eine Lösung von 2,5 g Triolein in 7 ml Propanol unter Druck in diese Lösung eingespritzt. Die so entstandene Emulsion wird bei −40 °C eingefroren und lyophilisiert.

22,4 g Mannit, 4 g Polyäthylenglykol MG 4000, 2 g Harnstoff, 7,47 g Natriumdesoxycholat, 0,82 g NaCl, 0,2 g Natriumazid, 2,71 g Tris und 0,4 g Tris.HCl werden gemahlen und innig miteinander gemischt. Hierzu wird die zerkleinerte Trockenemulsion gegeben und homogen vermischt.

90 mg dieses pulverförmigen Reagens werden in 2 ml destilliertes Wasser gegeben und nach dem Lösen mit 100 µl Probe (Serum) versetzt. Die Reaktion wird bei 340 (385) nmHg photometrisch verfolgt.

Die Tröpfchengrössenverteilung belastet und unbelastet (vgl. Beispiel 2) zeigt Fig. 4 der Zeichnung.

Beispiel 10

In 1000 ml destilliertem Wasser werden gelöst: 75 g Mannit, 25 g Polyäthylenglykol MG 4000, 20 g Taurodesoxycholat, 55 mMol HEPES-Puffer, pH 6,8, 0,3 g Calciumchlorid und 9 mg Colipase. Zu dieser Lösung wird 1 g Triolein, gelöst, eingespritzt (unter Rühren). Von dieser Emulsion werden je 1 ml in Flaschen abgefüllt und lyophilisiert.

Das Lyophilisat wird in 2,0 ml destilliertem Wasser gelöst. Die Lipasebestimmung wird durch Zugabe von 100 µl Serum und photometrische Messung bei 340 oder 365 nm durchgeführt.

Bei einer Lagerung des Lyophilisats bei 4 °C und 35 °C wurde nach drei Wochen bei Auflösen in bidestilliertem Wasser kein Unterschied in der Anfangsextinktion und im Testverlauf festgestellt.

| | |
|---|---|
| HEPES = | 2-[4-(2-Hydroxyäthyl)-piperazin-(1)]-äthansulfonsäure. |
| Tris-Puffer = | Tris (hydroxymethyl) amino-methan. |
| Bicine = | N-N-Bis(2-hydroxyethyl) glycin. |
| Taps-Puffer = | N-Tris (hydroxymethyl)methyl-3-aminopropansulfonsäure. |

**Patentansprüche**

1. Rekonstituierbares Trockenreagens zur insbesondere turbidimetrischen Bestimmung der Lipase, welches bei Wasserzugabe eine Emulsion bildet, mit einem Gehalt an Substratöl, Schutzkolloid, Emulgator und Aktivator, dadurch gekennzeichnet, dass es
0,2 bis 10 Gew.-% flüssiges Triglycerid,
20 bis 90 Gew.-% Schutzkolloid,
5 bis 60 Gew.-% Gallensäurealkalisalz,
0,001 bis 0,1 Gew.-% Colipase,
0,1 bis 2,0 Gew.-% Konservierungsmittel,
5 bis 20 Gew.-% Harnstoff,
3 bis 50 Gew.-% Puffersubstanz für pH 6,0 bis 10,5 und 0,5 bis 5 Gew.-% Aktivator
enthält.

2. Reagens nach Anspruch 1, dadurch gekennzeichnet, dass die Fettsäurereste des Triglycerids 8 bis 20 C-Atome und 1 bis 8 Kohlenstoff-Kohlenstoff-Doppelbindungen aufweisen.

3. Reagens nach Anspruch 1 oder 2, dadurch gekennzeichnet, dass es 0,2 bis 2,0 Gew.-% Triglycerid enthält.

4. Reagens nach einem der vorhergehenden Ansprüche, dadurch gekennzeichnet, dass das Schutzkolloid aus einer oder mehreren Polyhydroxyverbindungen besteht.

5. Reagens nach Anspruch 4, dadurch gekennzeichnet, dass die Polyhydroxyverbindung eine monomere oder polymere Pentose oder Hexose mit 1 bis 10 Pentose- bzw. Hexose-Einheiten im Molekül oder/und ein fester Polyäthylenglykol ist.

6. Reagens nach einem der vorhergehenden Ansprüche, dadurch gekennzeichnet, dass es 50 bis 70 Gew.-% Schutzkolloid enthält.

7. Reagens nach einem der vorhergehenden Ansprüche, dadurch gekennzeichnet, dass es 10 bis 15 Gew.-% Gallensäuresalz enthält.

8. Reagens nach einem der vorhergehenden Ansprüche, dadurch gekennzeichnet, dass es 0,003 bis 0,01 Gew.-% Colipase enthält.

9. Reagens nach einem der vorhergehenden Ansprüche, dadurch gekennzeichnet, dass es als Konservierungsmittel ein Alkaliazid enthält.

10. Reagens nach einem der vorhergehenden Ansprüche, dadurch gekennzeichnet, dass es 10 bis 15 Gew.-% Harnstoff enthält.

11. Reagens nach einem der vorhergehenden Ansprüche, dadurch gekennzeichnet, dass es 3 bis 10 Gew.-% Puffersubstanz enthält.

12. Reagens nach einem der vorhergehenden Ansprüche, dadurch gekennzeichnet, dass es als Puffersubstanz Hepes-Puffer, Tabs-Puffer, Good-Puffer, Tris-Puffer, Triäthanolamin-Puffer oder Diäthanolamin-Puffer enthält.

13. Reagens nach einem der vorhergehenden Ansprüche, dadurch gekennzeichnet, dass es als Aktivator ein Natrium-, Calcium- oder Magnesiumchlorid enthält.

14. Verfahren zur Herstellung des Trockenreagens nach einem der Ansprüche 1 bis 13, dadurch gekennzeichnet, dass man eine wässrige Emulsion des Triglycerids herstellt, welche Gallensäuresalz, Colipase, mindestens 10 Gew.-% Schutzkolloid, mindestens einen Teil des Konservierungsmittels und gegebenenfalls Aktivator enthält, die Emulsion lyophilisiert und dem Lyophilisat Puffersubstanz, Harnstoff und gegebenenfalls restliches Schutzkolloid und Konservierungsmittel sowie gegebenenfalls weiteren Aktivator zumischt.

15. Verfahren nach Anspruch 14, dadurch gekennzeichnet, dass man in eine Lösung von Schutzkolloid Gallensäuresalz, Colipase und gegebenenfalls Konservierungsmittel und Aktivator unter Rühren eine Lösung des Triglycerids in einem flüchtigen organischen Lösungsmittel in feinem Strahl einspritzt.

**Claims**

1. Reconstitutable dry reagent especially for the turbidimetric determination of lipase, which forms an emulsion upon the addition of water, with a content of substrate oil, protective colloid, emulsifier and activator, characterised in that it contains:
0.2 to 10 wt.% liquid triglyceride,
20 to 90 wt.% protective colloid,
5 to 60 wt.% alkali metal salt of bile acid,
0.001 to 0.1 wt.% colipase,
0.1 to 2.0 wt.% preservation agent,
5 to 20 wt.% urea,
3 to 50 wt.% buffer substance for pH 6.0 to 10.5 and
0.5 to 5 wt.% activator.

2. Reagent according to claim 1, characterised in that the fatty acid residue of the triglyceride has 8 to 20 C-atoms and 1 to 8 carbon-carbon double bonds.

3. Reagent according to claim 1 or 2, characterised in that it contains 0.2 to 2.0 wt.% triglyceride.

4. Reagent according to one of the preceding claims, characterised in that the protective colloid consists of one or more polyhydroxy compounds.

5. Reagent according to claim 4, characterised in that the polyhydroxy compound is a monomeric or polymeric pentose or hexose with 1 to 10 pentose or hexose units in the molecule and/or a solid polyethylene glycol.

6. Reagent according to one of the preceding claims, characterised in that it contains 50 to 70 wt.% of protective colloid.

7. Reagent according to one of the preceding claims, characterised in that it contains 10 to 15 wt.% of bile acid salt.

8. Reagent according to one of the preceding claims, characterised in that it contains 0.003 to 0.01 wt.% colipase.

9. Reagent according to one of the preceding claims, characterised in that it contains an alkali metal azide as preservation agent.

10. Reagent according to one of the preceding claims, characterised in that it contains 10 to 15 wt.% urea.

11. Reagent according to one of the preceding claims, characterised in that it contains 3 to 10 wt.% buffer substance.

12. Reagent according to one of the preceding claims, characterised in that as buffer substance it contains hepes buffer, tabs buffer, Good buffer, tris buffer, triethanolamine buffer or diethanolamine buffer.

13. Reagent according to one of the preceding claims, characterised in that as activator it contains sodium, calcium or magnesium chloride.

14. Process for the preparation of a dry reagent according to one of claims 1 to 13, characterised in that one prepares an aqueous emulsion of the triglyceride which contains bile acid salt, colipase, at least 10 wt.% protective colloid, at least a part of the preservation agent and possibly activator, lyophilises the emulsion and mixes with the lyophilisate buffer substance, urea and possibly remaining protective colloid and preservation agent, as well as possibly further activator.

15. Process according to claim 14, characterised in that one injects, with stirring, a solution of the triglyceride in a volatile organic solvent in a fine stream into a solution of protective colloid, bile acid salt, colipase and possibly preservation agent and activator.

**Revendications**

1. Réactif sec reconstituable, en particulier pour la détermination turbidimétrique de la lipase, qui forme par addition d'eau une émulsion, avec une teneur en huile de substrat, colloïde protecteur, émulsionnant et activateur, caractérisé en ce qu'il contient:
0,2 à 10% en poids de triglycéride liquide,
20 à 90% en poids de colloïde protecteur,
5 à 60% en poids de sel alcalin d'acide biliaire,
0,001 à 0,1% en poids de colipase,
0,1 à 2,0% en poids d'agent conservateur,
5 à 20% en poids d'urée,
3 à 50% en poids de substance tampon pour un pH de 6,0 à 10,5 et
0,5 à 5% en poids d'un activateur.

2. Réactif suivant la revendication 1, caractérisé en ce que les radicaux acide gras du triglycéride présentent 8 à 20 atomes de carbone, et 1 à 8 doubles liaisons carbone-carbone.

3. Réactif suivant la revendication 1 ou 2, caractérisé en ce qu'il contient 0,2 à 2,0% en poids de triglycéride.

4. Réactif suivant l'une des revendications précédentes, caractérisé en ce que le colloïde protecteur est constitué d'un ou plusieurs composés polyhydroxylés.

5. Réactif suivant la revendication 4, caractérisé en ce que le composé polyhydroxylé est un pentose ou un hexose monomère ou polymère avec 1 à 10 motifs pentose ou hexose dans sa molécule ou/et un polyéthylèneglycol solide.

6. Réactif suivant l'une des revendications précédentes, caractérisé en ce qu'il contient 50 à 70% en poids de colloïde protecteur.

7. Réactif suivant l'une des revendications précédentes, caractérisé en ce qu'il contient 10 à 15% en poids de sel d'acide biliaire.

8. Réactif suivant l'une des revendications précédentes, caractérisé en ce qu'il contient 0,003 à 0,01% en poids de colipase.

9. Réactif suivant l'une des revendications précédentes, caractérisé en ce qu'il contient comme agent de conservation un azide alcalin.

10. Réactif suivant l'une des revendications précédentes, caractérisé en ce qu'il contient 10 à 15% en poids d'urée.

11. Réactif suivant l'une des revendications précédentes, caractérisé en ce qu'il contient 3 à 10% en poids de substance tampon.

12. Réactif suivant l'une des revendications précédentes, caractérisé en ce qu'il contient comme substance tampon du tampon Hepes, du tampon Tabs, du tampon Good, du tampon Tris, du tampon à la triéthanolamine ou du tampon à la diéthanolamine.

13. Réactif suivant l'une des revendications précédentes, caractérisé en ce qu'il contient comme activateur un chlorure de sodium, de calcium ou de magnésium.

14. Procédé de préparation du réactif sec suivant l'une des revendications 1 à 13, caractérisé en ce qu'on prépare une émulsion aqueuse du triglycéride qui contient du sel d'acide biliaire, de la colipase, au moins 1o% en poids de colloïde protecteur, au moins une partie de l'agent de conservation et le cas échéant de l'activateur, en ce qu'on lyophilise l'émulsion et en ce qu'on mélange au lyophilisat de la substance tampon, de l'urée et le cas échéant le reste de colloïde protecteur et de l'agent de conservation, et le cas échéant encore de l'activateur.

15. Procédé suivant la revendication 14, caractérisé en ce qu'on injecte en un fin jet dans une solution de colloïde protecteur de sel d'acide biliaire, de colipase et le cas échéant d'agent de conservation et d'activateur, en agitant, une solution du triglycéride dans un solvant organique volatil.

FIG. 1

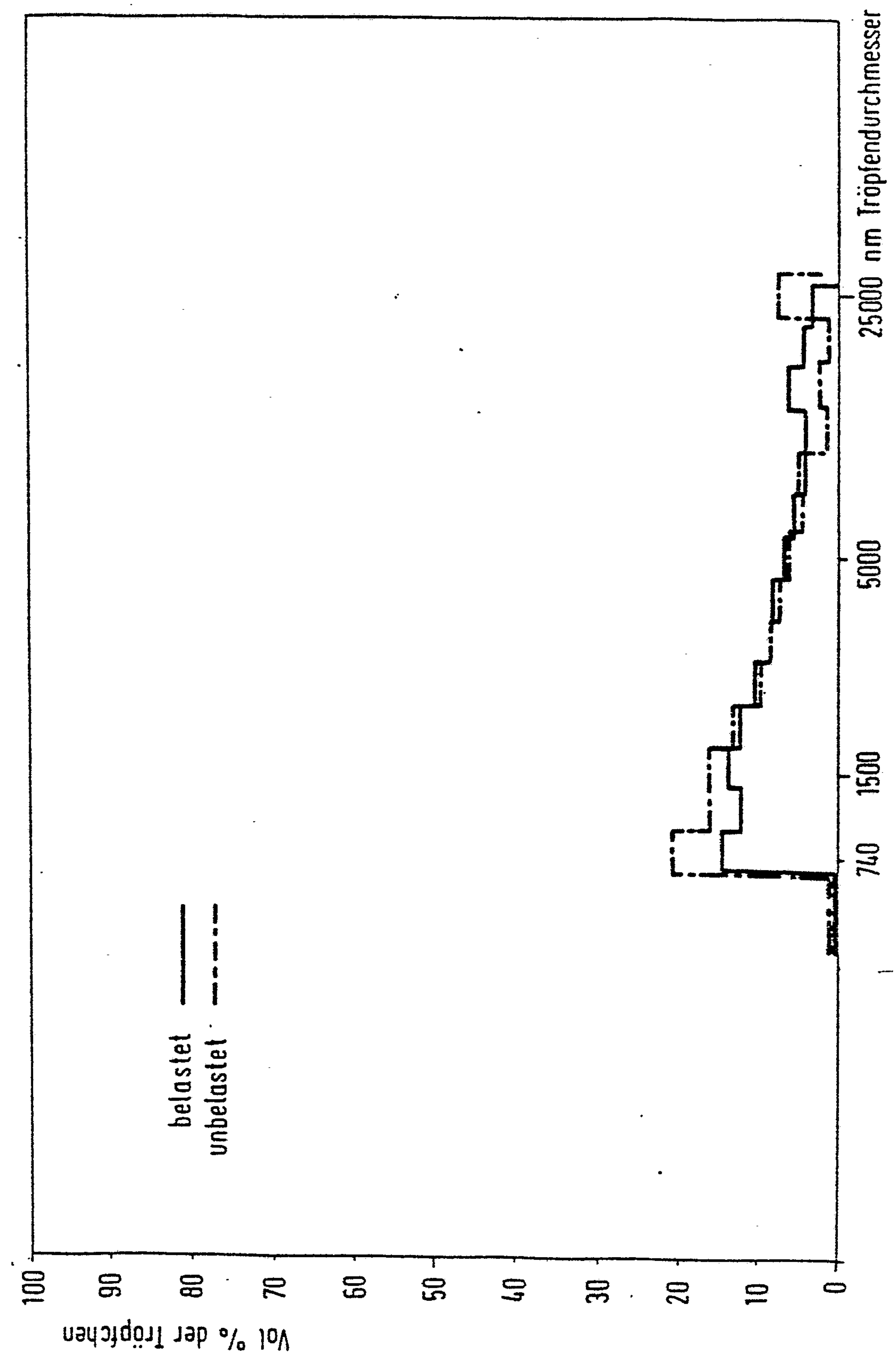
belastet
unbelastet
Vol % der Tröpfchen
0
10
20
30
40
50
60
70
80
90
100
740
1500
5000
25000 nm Tröpfendurchmesser

FIG. 2

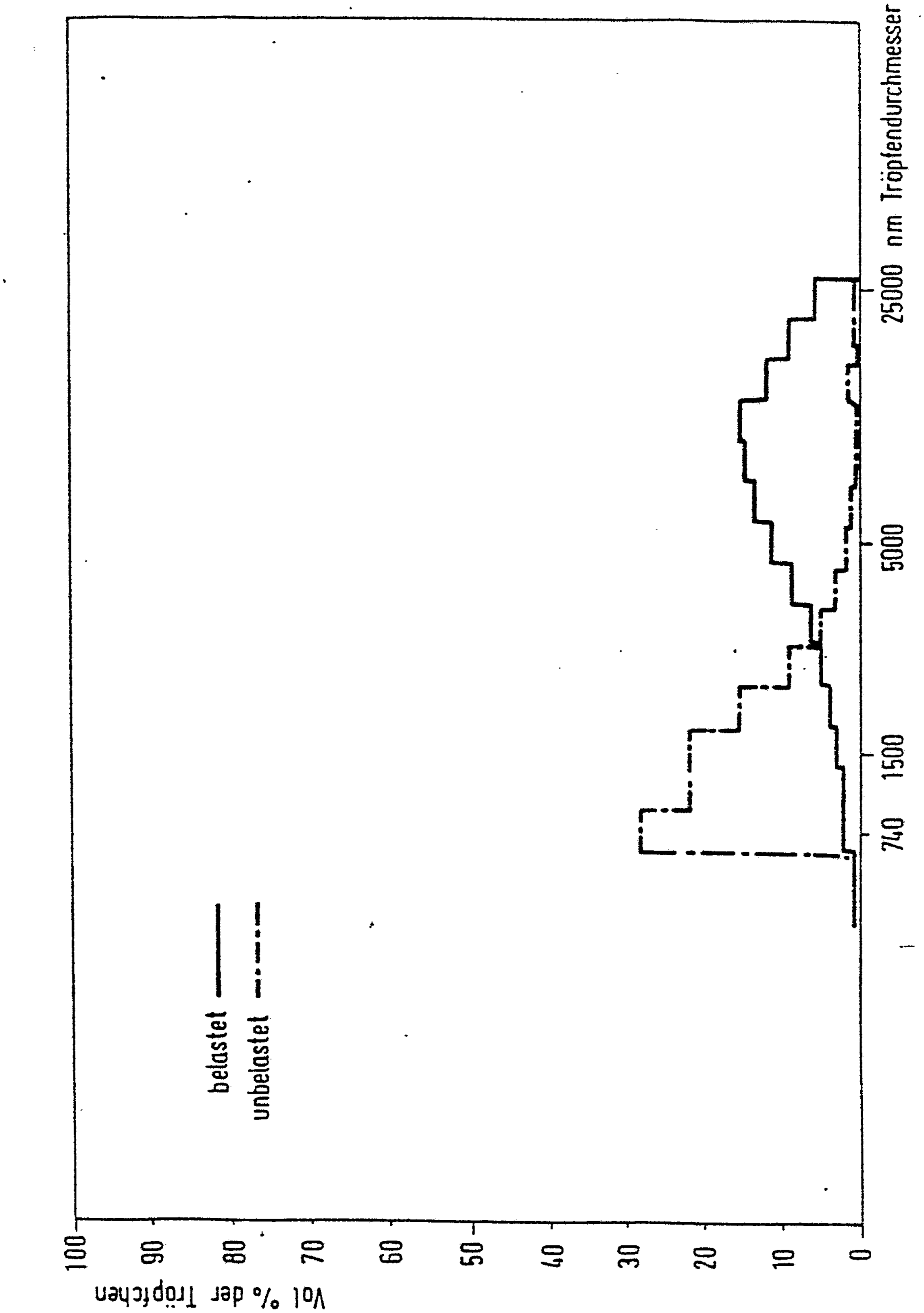
belastet
unbelastet
Vol % der Tröpfchen
100
90
80
70
60
50
40
30
20
10
0
740
1500
5000
25000 nm Tröpfendurchmesser

F I G. 3

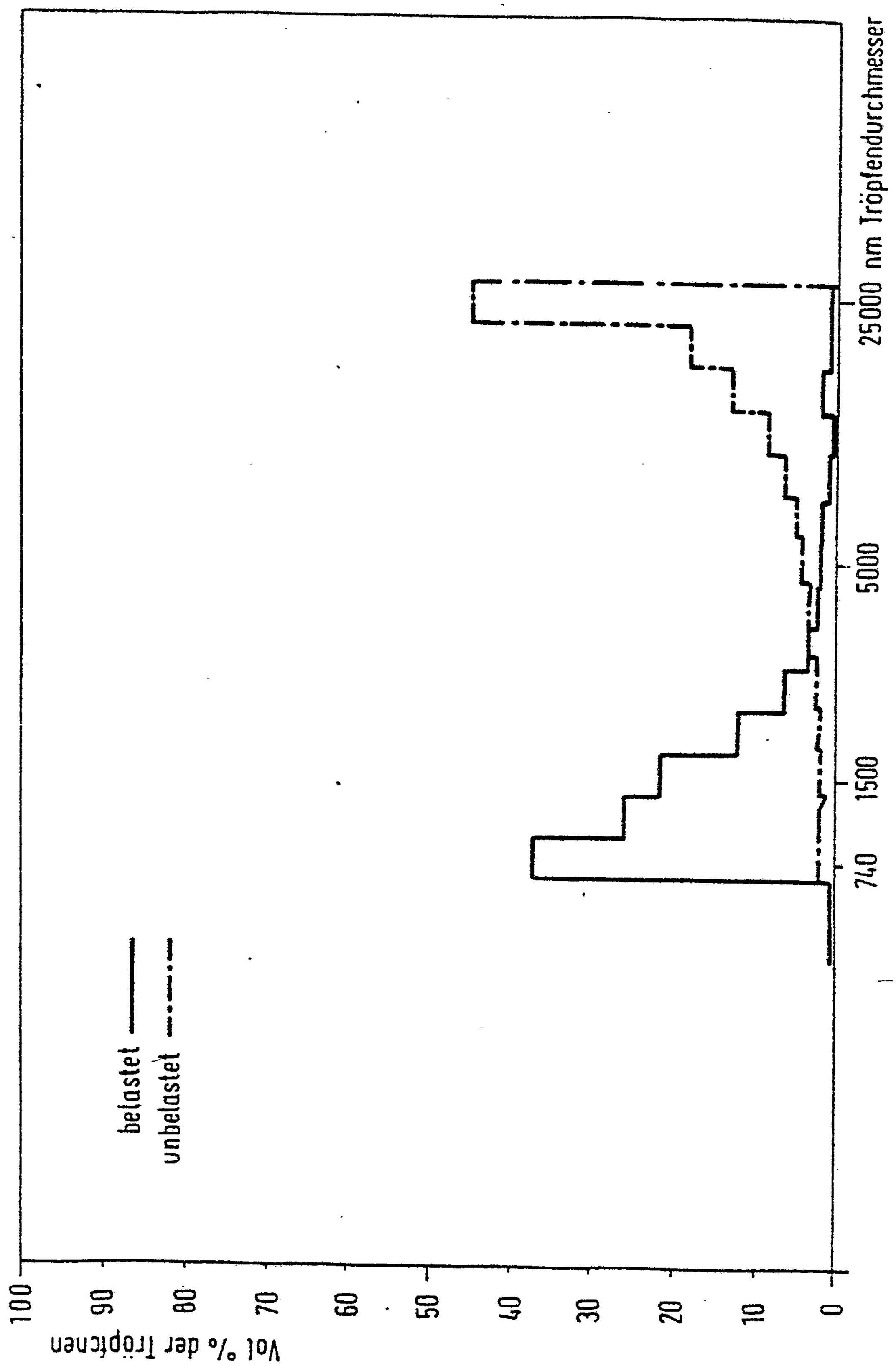
belastet
unbelastet
Vol % der Tröpfchen
100
90
80
70
60
50
40
30
20
10
0
740
1500
5000
25000 nm Tröpfendurchmesser

FIG. 4

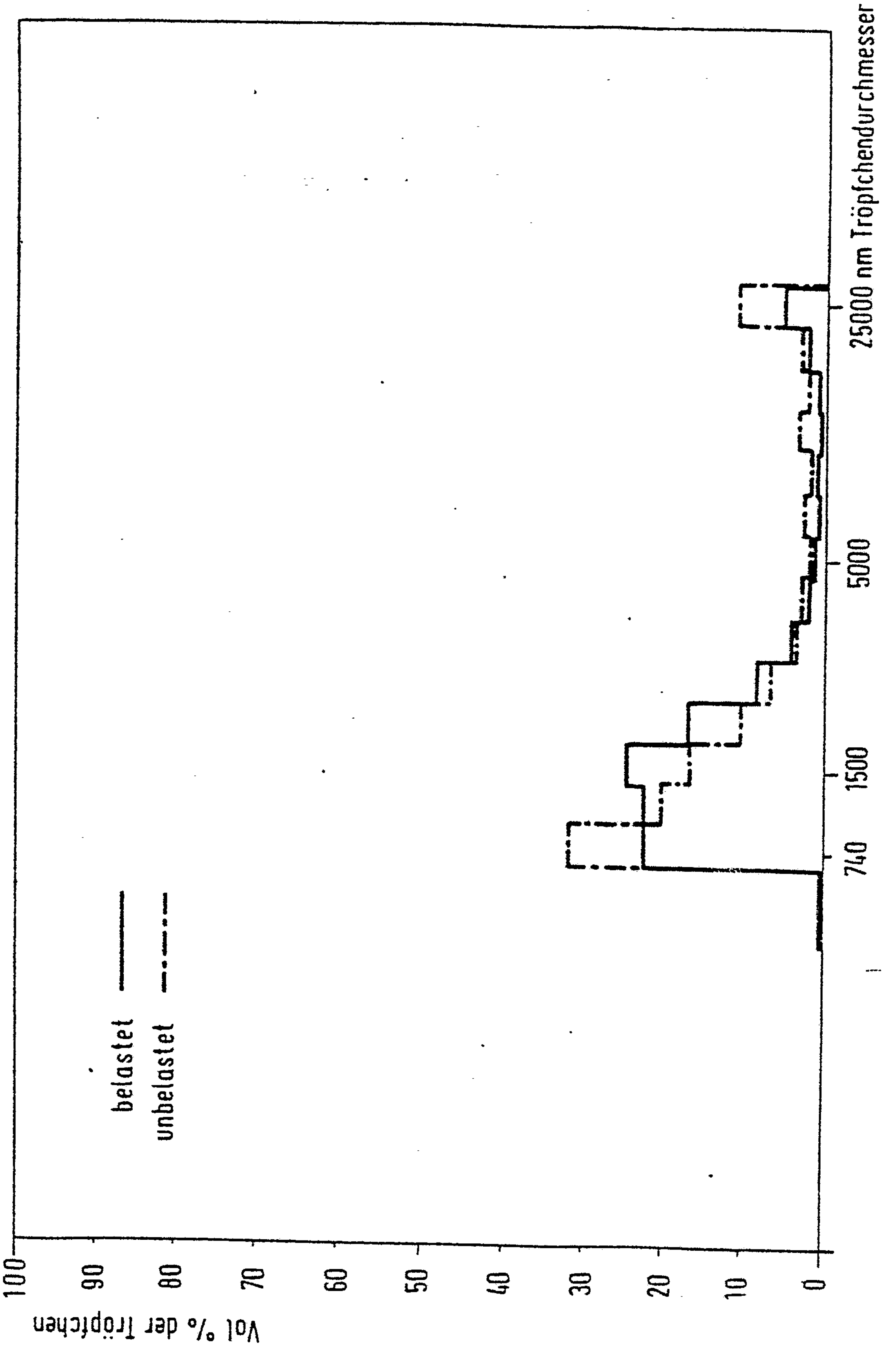
belastet
unbelastet
100
90
80
70
60
50
40
30
20
10
0
Vol % der Tröpfchen
740
1500
5000
25000 nm Tröpfchendurchmesser